# EUROPÄISCHE PATENTANMELDUNG

(11) **EP 1 868 005 A2**
(43) Veröffentlichungstag der Anmeldung: **19.12.2007**
(21) Anmeldenummer: 07104990.2
(22) Anmeldetag: 27.03.2007
(51) Int. Cl.: G01R 33/02

(54) **Terahertz imaging**

(30) Priorität: 24.05.2006 EP 06010634
(71) Anmelder: BrainLAB AG, 85622 Feldkirchen (DE)
(72) Erfinder: Goldbach, Günter, 85457, Wörth/Wifling (DE)
(74) Vertreter: Schwabe - Sandmair - Marx

(57) **Zusammenfassung**

Die vorliegende Erfindung bezieht sich auf ein Verfahren zur Untersuchung eines Körpers (2), wobei die von dem Körper (2) ausgesandte und/oder reflektierte Strahlung im Terahertz-Frequenzbereich, insbesondere zwischen 0,1 und 30 Terahertz, mit einem Sensor (1) erfasst und von einer Recheneinheit (3) ausgewertet wird, um Informationen über den Körper (2) zu erhalten; sowie auf eine Vorrichtung zur Untersuchung eines Körpers (2) mit einem Terahertz-Sensor (1), insbesondere einer Terahertz-Kaznera zum Erfassen von an einem Körper (2) oder einer Körperstruktur reflektierter oder durch einen Körper (2) oder eine Körperstruktur transmittierter oder von einem Körper (2) oder einer Körperstruktur emittierter Terahertz-Strahlung und mit einer Recheneinheit (3), wie einem Tracking-System, zum Auswerten der erfassten Terahertz-Strahlung, insbesondere zum Gewinnen von Informationen über den Körper (2) oder die Körperstruktur, wobei die Recheneinheit (3) mit dem Terahertz-Sensor (1) verbunden ist.

## Beschreibung

Die vorliegende Erfindung betrifft ein Verfahren und eine Vorrichtung zur Untersuchung eines Körpers, insbesondere ein im medizinischen Bereich angewandtes bildgebendes Verfahren oder eine bildgebende Vorrichtung, wobei der Körper mittels Strahlung im Terahertz-Frequenzbercich untersucht wird, um Informationen über den Körper, insbesondere über dessen Form, Art oder Position zu erhalten.

Bekannte bildgebende medizinische Systeme können gesundheitsschädlich sein, wie Röntgensysteme, ungenau sein, wie Ultraschallsysteme, können Weichteile nicht befriedigend darstellen, wie Computertomographiesysteme, oder können Knochen nicht ausreichend gut darstellen, wie Magnetresonanzsysteme.

Es ist eine Aufgabe der vorliegenden Erfindung ein Verfahren und eine Vorrichtung zur Untersuchung eines Körpers vorzuschlagen, welche eine genaue, hochauflösende und kostengünstige Untersuchung eines Körpers gewährleisten.

Bei dem erfindungsgemäßen Verfahren zur Untersuchung eines Körpers oder eines Körperteils oder einer Körperstruktur eines Patienten wird die von dem Körper oder Körperteil oder der Körperstruktur ausgesandte oder reflektierte Strahlung im Terahertz-Frequenzbereich mit einem Sensor oder einem Detektor erfasst, wobei unter dem verwendeten Terahertz-Frequenzbereich eine Strahlung mit einer Frequenz zwischen 0,1 und 30 THz zu verstehen ist. Unter der von dem Körper ausgesandten Strahlung im Terahertz-Frequenzbereich kann sowohl eine Eigenemission des Körpers im Terahertz-Frequenzbereich als auch eine Durchstrahlung oder Transmission des Körpers mit Strahlung im Terahertz-Frequenzbereich verstanden werden. Die ausgesandte oder reflektierte Strahlung des Körpers oder Körperteils oder der Körperstruktur kann mit einem Terahertz-Sensor erfasst und von einer Recheneinheit ausgewertet oder verarbeitet werden, um Informationen über den Körper oder die Körperstruktur zu erhalten.

Es kann zum Beispiel die mit dem Terallertz-Sensor erfasste Strahlung so ausgewertet werden, dass Informationen über die Beschaffenheit, die Art, die Zusammensetzung, das Material, die Form, die Struktur, den Zustand, die Temperatur oder die Position der Oberfläche oder des Inneren des Körpers oder des Körperteils oder der Körperstruktur erhalten werden.

Aus den erhaltenen Informationen über den Körper kann zum Beispiel ein Registrierungsvorgang, insbesondere ein automatischer Registrierungsvorgang, des Körpers oder des Körperteils oder der Körperstruktur durchgeführt werden, indem zum Beispiel Landmarkenpunkte des Körpers identifiziert und die Landmarken Raumpositionen zugeordnet werden, oder indem Aufnahmen mit einer Kamera aus verschiedenen bekannten Positionen durchgerührt und die Aufnahmen weiterverarbeitet werden bis eine Registrierung des Körpers oder des Körperteils oder der Körperstruktur erfolgt ist.

Die mittels der Teraheriz-Strahlung ermittelten Informationen über den Körper können auch mit weiteren Informationen kombiniert werden. So können zum Beispiel mehrere Aufnahmen des Körpers oder des Körperteils mittels der Terahertz-Strahlung durchgeführt werden, wobei die verwendete Terahertz-Strahlung dieselbe Frequenz oder denselben Frequenzbereich oder eine unterschiedliche Frequenz oder einen unterschiedlichen Frequenzbereich aufweisen kann, so dass zum Beispiel derselbe Körper oder dieselben Körperstrukturen aus unterschiedlichen Positionen aufgenommen und miteinander kombiniert werden können oder unterschiedliche Teile eines Körpers, wie die Oberfläche oder das Innere eines Körpers, mittels verschiedener Frequenzen oder Frequenzbereiche aufgenommen und miteinander kombiniert werden können. In Abhängigkeit von der gewählten Frequenz kann die Strahlung nicht oder nur gering in die Oberfläche des Körpers eindringen und wird z.B. an der Oberfläche oder nahe der Oberfläche reflektiert oder kann tief in den Körper eindringen oder den Körper durchdringen oder z.B. auch Kleidung durchdringen. Auch können die mittels der Terahertz-Strahlung ermittelten Informationen über den Körper oder die Körperstruktur oder das Körperteil mittels eines Bildfusionsvorganges mit Informationen über denselben Körper oder dieselbe Körperstruktur oder dasselbe Körperteil kombiniert werden, die zum Beispiel mit einem weiteren bildgebenden Verfahren, wie einem Röntgenverfahren, einem Magnetresonanzverfamen, einem Computertomographieverfahren, einem Ultraschallverfahren, einem Positron-Emissions-Tomographieverfahren (PET-Verfahren) oder einem Single Photon Emission Computed Tomography-Verfahren (SPECT-Verfahren) ermittelt werden. Vorzugsweise werden die aus der erfassten Terahertz-Strahlung ermittelten Informationen über den Körper mit zwei- oder dreidimensionalen Informationen über den Körper, die zum Beispiel mittels Terahertz-Strahlung oder eines anderen bildgebenden Verfahrens ermittelt wurden, kombiniert.

Der Terahertz-Sensor kann zum Beispiel von dem Körperteil oder der Körperstruktur emittierte Strahlung im Terahertz-Frequenzbererch erfassen. Auch kann der untersuchte Körper zum Beispiel mit Terahertz-Strahlung bestrahlt oder angestrahlt werden, so dass die an dem Körper reflektierte, zum Beispiel die an der Oberfläche des Körpers oder Nahe der Oberfläche des Körpers reflektierte Strahlung von einem Terahertz-Sensor, insbesondere einem Terahertz-Sensor-Feld, welches bevorzugt um den Körper oder das Körperteil angeordnet ist, erfasst wird. Auch kann Terahertz-Strahlung mit dem Terahertz-Sensor erfasst werden, welche durch den Körper durchgestrahlt oder transmittiert wird, wobei der Sensor vorzugsweise gegenüber der Strahlungsquelle angeordnet ist. In Abhängigkeit von der Beschaffenheit, der Art, der Zusammensetzung, dem Material, der Form, der Struktur, des Zustands, der Temperatur oder der Position des Körpers oder der Körperstruktur oder des Körperteils wird unterschiedlich viel Terahertz-Strahlung durch den Körper transmittiert, oder von dem Körper reflektiert, oder von dem Körper emitiert, oder die Terahertz-Strahlung wird unterschiedlich stark gedämpft oder absorbiert, so dass aus der erfassten Terahertz-Strahlung Informationen über die Oberfläche oder das Innere des Körpers gewonnen werden können.

Basierend auf den ermittelten Informationen über den Körper oder das Körperteil kann auch ein Instrument, wie ein Mikroskop oder ein Endoskop, auf dem zum Beispiel aktive oder passive Marker angeordnet sind, navigiert werden.

Es können zum Beispiel Eigenschaften über den Körper oder das Körperteil, wie die Beschaffenheit, die Form, die Struktur, der Zustand oder die Position des Körpers oder der Oberfläche des Körpers ermittelt werden, indem der Körper aus einer vorzugsweise bekannten Entfernung mit Terahertz-Strahlung bestrahlt wird und die an dem Körper oder der Oberfläche des Körpers reflektierte Strahlung mit einem Sensor erfasst oder detektiert wird, wobei aus der Laufzeit der Terahertz-Strahlung oder des Terahertz-Signals zum Beispiel auf die Form oder die Position oder die Entfernung des Körpers oder der Oberfläche geschlossen werden kann,

Auch kann die mit dem Sensor erfasste Terahertz-Strahlung oder das Terahertz-Signal so ausgewertet werden, dass zum Beispiel mittels einer Fourier-Transformation das Spektrum oder eine Frequenzbereichsdarstellung der Strahlung oder des Signals bestimmt wird und aus den spektralen Eigenschaften auf Eigenschaften des Körpers oder des Körperteils, welches die Strahlung emittiert oder reflektiert oder transmittiert hat, geschlossen wird. Die ermittelte spektrale Darstellung der erfassten Terahertz-Strahlung kann zum Beispiel mit Spektren bekannter Materialien oder Formen oder Zuständen oder Temperaturen verglichen werden, wobei aus dem Vergleich die Eigenschaften des untersuchten Körpers oder Körperteils ermittelt werden können,

Bevorzugt können auch charakteristische Frequenzen des erfassten Terahertz-Signals, wie Frequenzen maximaler oder minimaler Absorption, Reflexion oder Transmission mit charakteristischen Frequenzen, wie Resonanzfrequenzen oder maximalen oder minimalen Absorptions-, Transmissions- oder Reflexionsfrequenzen bekannter Körper oder Körperstrukturen, die zum Beispiel zuvor ermittelt oder gespeichert worden sind, verglichen werden und aus dem Vergleich der Frequenzen die Beschaffenheit, die Art, die Zusammensetzung, das Material, die Form, die Struktur, der Zustand, die Temperatur oder die Position des Körpers oder der Oberfläche oder des Inneren des Körpers ermittelt werden.

Insbesondere können mittels der Terahertz-Strahlung ein Körper oder Körperteile, wie ein Kopf, ein Gesicht, ein Arm, ein Kiefer, ein Gebiss oder eine Hand untersucht werden und Körperstrukturen, wie Gewebe, Knochen bzw. Knochenstrukturen, Gefäße, Bänder, Sehnen, Zähne oder Haut eines Patienten untersucht werden. Zum Beispiel könnte in der craniellen Applikation nicht nur die Außenkontur bzw. Oberfläche des Gesichtes oder Kopfes dargestellt werden, sondern detaillierte Informationen über die Position von markanten Knochenstrukturen unter der Haut bestimmt werden. Auch können zum Beispiel Tumore am offen liegenden Gehirn erkannt werden, indem zum Beispiel während einer Operation mittels eines spektralen Terahertz-Sensors vom Gehirn emittierte Terahertz-Strahlung erfasst wird, um detaillierte Informationen über die Art des Gewebes zu erhalten. So kann beispielsweise durch Überlagerung im Mikroskop anhand spektraler Informationen erkannt werden, welches Gewebe Tumor ist, und welches nicht. Auch können mittels der erfassten Terahertz-Strahlung oberflächennahe Tumore, wie Hautkrebs, zum Beispiel anhand der unterschiedlichen spektralen Charakteristik von krankern und gesundem Gewebe, erkannt werden, so dass in Kombination mit einem Tracking-System zur Gewinnung von dreidimensionalen Informationen über die Position der Tumore eine gezielte Behandlung, zum Beispiel durch Unterspritzung oder Resektion der Tumore, ermöglicht werden kann. Des Weiteren kann zum Beispiel basierend auf Terahertz-Bildern in der Handchirurgie ein Navigationsvorgang durchgeführt werden. Hierbei können vor, während oder nach einer Operation Informationen bezüglich der Temperatur, wie bei Schwellungen und Gefäßverletzungen, der Art und der Position von Gefäßen und Bändern ermittelt werden. In zahnmedizinischen Applikationen kann mittels der Terahertz-Strahlung zum Beispiel die Dicke des Zahnschmelzes, der innere Zustand der Zähne oder die Form der Zähne festgestellt werden oder es kann Karies oder Paradonthose festgestellt werden,

Bevorzugt wird zur Untersuchung des Körpers oder der Körperstruktur Terahertz-Strahlung in einem Frequenzbereich zwischen 0,1 und 5 THz verwendet, wobei vorzugsweise die spektralen Bereiche zwischen 0,1 und 0,6 THz und zwischen 0,5 und 2 THz verwendet werden. Insbesondere kann auch Terahertz-Strahlung in einem Bereich um 1,6 THz oder in einem Bereich um 2,5 THz oder in einem weiten breitbandigen Bereich um 3 THz verwendet werden.

Die Erfindung bezieht sich des Weiteren auf ein Computerprogramm, weiches, wenn es in einen Computer geladen wird oder auf einem Computer läuft ein wie oben beschriebenes Verfahren durchführt. Weiterhin bezieht sich die Erfindung auf ein Programmspeichermedium oder ein Computerprogrammprodukt mit einem solchen Programm.

Die erfindungsgemäße Vorrichtung zur Untersuchung eines Körpers umfasst einen Terahertz-Sensor oder einen Terahertz-Detektor, insbesondere eine Terahertz-Kamera, welcher von einem Körper oder einer Körperstruktur reflektierte oder durch einen Körper oder eine Körperstruktur transmittierte oder von einem Körper oder einer Körperstruktur emitierte Teraheriz-Strahlung erfassen kann.

Die erfindungsgemäße Vorrichtung umfasst weiter eine Recheneinheit, wie ein Tracking-System, das mit dem Terahertz-Sensor drahtgebunden oder drahtlos verbunden ist, wodurch die erfasste Terahertz-Strahlung oder die erfassten Daten an die Recheneinheit übertragen werden können, in welcher die erfasste Terahertz-Strahlung ausgewertet oder weiterverarbeitet werden kann. In der Recheneinheit kann die erfasste Terahertz-Strahlung ausgewertet werden, um Informationen über den Körper oder die Körperstruktur zu erhalten. Vorzugsweise kann das mittels des Terahertz-Sensors erfasste Terahertz-Signal analysiert werden, wie zum Beispiel mittels einer Fourier-Transmformation in den Frequenzbereich transformiert werden, so dass zum Beispiel aus dem Spektrum des erfassten Terahertz-Signals charakteristischer Frequenzen, wie Absorptions- oder Resonanzfrequenzen ermittelt werden können.

Die erfindungsgemäße Vorrichtung kann weiter auch eine Teraheriz-Strahlungsquelle, insbesondere Terahertz-Lampen umfassen, welche Terahertz-Strahlung aussenden kann, um den Körper oder die Körperstruktur mit Terahertz-Strahlung zu bestrahlen oder zu durchstrahlen, wobei die Terahertz-Strahlungsquelle mit dem Terahertz-Sensor oder mit der Recheneinheit verbunden sein kann. Die von der Terahertz-Strahlungsquelle ausgesandte Strahlung kann zum Beispiel an der zu untersuchenden Körperstruktur oder dem zu untersuchenden Körperteil reflektiert werden oder kann durch den Körper oder die Körperstruktur transmittiert werden, so dass die reflektierte oder transmittierte Strahlung von dem Terahertz-Sensor erfasst werden kann. Insbesondere kann die Terahertz-Strahlungsquelle an einem bekannten Ort angeordnet sein, oder kann mit Markern versehen sein, so dass die Position der Terahertz-Strahlungsquelle zum Beispiel von einem Tracking-System ermittelt werden kann. Wird zum Beispiel eine Transmissionsmessung eines Körpers durchgeführt, so befindet sich die Terahertz-Strahlungsquelle vorzugsweise gegenüber des Terahertz-Sensars, so dass von der Terahertz-Strahlungsquelle ausgesandte Strahlung den Körper oder die Körperstruktur zumindest teilweise durchdringt und auf der gegenüberliegenden Seite von dem Terahertz-Sensor erfasst werden kann. Wird zum Beispiel eine Reflexionsmessung durchgeführt, so kann die Terahertz- Strahlungsquelle zum Beispiel an einem bekannten oder ermittelbaren Ort angeordnet sein, wobei der Terahertz-Sensor, zum Beispiel als Sensorfeld, um den Körper oder die Körperstruktur angeordnet sein kann, um die an dem Körper zumindest teilweise reflektierte Terahertz-Strahlung zu erfassen.

Die Vorrichtung der vorliegenden Erfindung kann auch eine Datenbank aufweisen, welche vorzugsweise mit einer Recheneinheit verbunden ist und in welcher charakteristische Informationen einer Mehrzahl von Körpern oder Körperstrukturen gespeichert sein kann. Insbesondere können charakteristische Frequenzen, wie Absorption- oder Resonanzfrequenzen, bestimmter Körperteile oder Körperstrukturen, wie Gewebe, Knochen, Gefäße, Bänder, Sehnen, Zähne oder Haut in der Datenbank gespeichert sein oder es können charakteristische als spektraler Fingerabdruck eines Körpers oder einer Körperstruktur dienende Spektren einer Mehrzahl von Körperteilen oder Körperstrukturen gespeichert sein. Vorzugsweise kann die Recheneinheit die mittels der erfassten Terahertz-Strahlung gewonnene Information über den Körper oder die Körperstruktur mit den in der Datenbank gespeicherten Informationen vergleichen, insbesondere einen Vergleich der charakteristischen Frequenzen oder des Spektrums bzw. Frequenzbereichs durchführen, und aus dem durchgeführten Vergleich Rückschlüsse auf die Beschaffenheit, die Art, die Zusammensetzung, das Material, die Form, die Struktur, den Zustand, die Temperatur oder die Position der Oberfläche oder des Inneren des Körpers ziehen. Vorzugsweise werden charakteristische Frequenzen des erfassten Terahertz-Signals oder das Spektrum des erfassten Terahertz-Signals mit den gespeicherten Informationen verglichen und zum Beispiel aus der Ähnlichkeit charakteristischer Frequenzen oder der Spektren geschlossen, dass ein bestimmtes Material oder eine bestimmte Struktur oder Temperatur des Körpers vorliegt.

Insbesondere kann die erfindungsgemäße Vorrichtung auch eine Datenausgabevorrichtung, insbesondere einen Bildschirm, aufweisen, der die ermittelten Informationen über den Körper als Zahlenwerte oder als Bild anzeigen kann oder fusionierte oder registrierte Körperstrukturen darstellen kann. Zudem kann auch eine Dateneingabevoirichtung mit der Recheneinheit oder der Datenbank verbunden sein, insbesondere eine Tastatur oder ein Scanner wie eine Röntgenvorrichtung, ein Computertomograph, ein Kernspintomograph, ein Ultraschalltomograph, ein Positron-Emissions-Tomograph oder ein Single Photon-Emission-Tomography-Tomograph. Mittels der Dateneingabevorrichtung können Informationen in die Recheneinheit oder in die Datenbank eingegeben werden, so dass die Informationen in der Datenbank abgespeichert werden können oder in der Recheneinheit zum Beispiel mit den mittels der Terahertz-Strahlung gewonnenen Informationen verglichen werden können oder verarbeitet werden können.

Vorzugsweise umfasst die Terahertz-Strahlungsquelle oder der Terahertz-Sensor einen elektronischen oder optischen Terahertz-Oszelator, wie einen Titan:Saphir-Laser.

Die Erfindung wird nachfolgend anhand bevorzugter Ausführungsbeispiele beschrieben werden. Es zeigen:
- Figur 1: eine Ausführungsform der vorliegenden Erfindung;
- Figur 2: ein exemplarisches Diagramm der erfassten Terahertz-Strahlung im Zeit- und im Frequenzbereich.

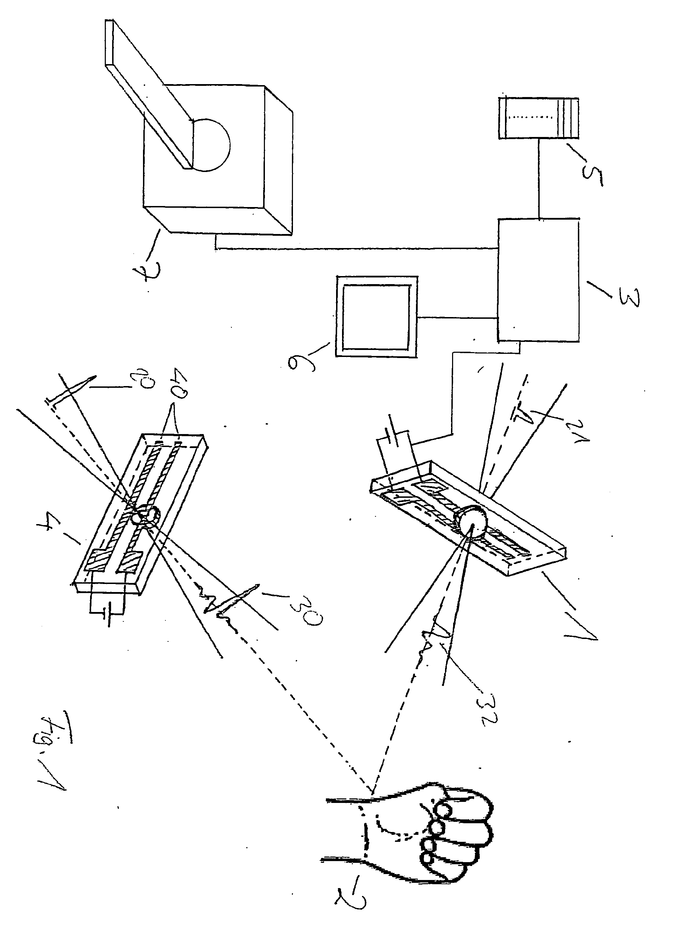

Figur 1 zeigt eine Ausführungsform der vorliegenden Erfindung, wobei als Körper 2 oder Körperteil eine Hand 2 mittels einer Teraheitz-Strahlungsquelle 4 bestrahlt oder angestrahlt wird. Die Terahertz-Strahlungsquelle 4 umfasst vorzugsweise einen modengekoppelten Titan:Saphir-Laser, welcher Pulse 20 von nur wenigen zehn Ferntosekunden Dauer emittieren kann. Mit diesen optischen Pulsen 20 können fotoleitende Dipolantennen 40 geschaltet werden, welche vorzugsweise aus Galliumarsenid bestehen, auf das zwei Metallstreifenleitungen aufgedampft worden sind. Die kurzen Laserpulse 20 erzeugen zwischen den Leitungen Ladungsträger, die durch ein an den Dipolantennen 40 angelegtes elektrisches Feld beschleunigt werden, woraus ein kurzer Stromimpuls resultiert, welcher einen in den Raum abgestrahlten Terahertz-Puls 30 erzeugt. Der Terahertz-Puls trifft in der vorliegenden Ausführungsform der Erfindung auf eine Hand 2 und wird an deren Oberfläche oder von Strukturen nahe der Oberfläche reflektiert, wobei der reflektierte Terahertz-Puls 32 von einem Terahertz-Sensor 1 bzw. Terahertz-Detektor erfasst wird. Der Terahertz-Sensor 1 kann wie in der vorliegenden Ausführungsform einen ähnlichen Aufbau wie die Terahertz-Strahlungsquelle 4 besitzen, wobei kein externes Feld angelegt sein muss oder kann andersartig, wie z.B. als rein elektronischer Sensor, ausgestaltet sein. Ein Schaltlaserpuls 21 erzeugt in dem Terahertz-Sensor 1 freie Ladungsträger, welche sich im elektrischen Feld der einlaufenden bzw. erfassten von der Hand 2 reflektierten Terahertz-Welle 32 bewegen, wodurch es zu einem kleinen Stromfluss kommt, welcher verstärkt und registriert werden kann. Der erzeugte Stromfluss kann an eine Recheneinheit 3, wie einen Computer, übertragen und dort weiterverarbeitet oder ausgewertet werden. Aus dem erzeugten Strom kann z.B. der zeitliche Verlauf der erfassten Terahertz-Strahlung 32 ermittelt werden sowie z.B, mittels einer Fourier-Transfbrmation das Spektrum oder die Frequenzbereichsdarstellung des erfassten Terahertz-Pulses 32 in der Recheneinheit 3 bestimmt werden. Die ermittelten Informationen können vorzugsweise auf der Datenausgabevorrichtung 6, wie einem Bildschirm, ausgegeben werden sowie mit in der Datenbank 5 gespeicherten Zeit- und Frequenzbereichsdarstellungen bekannter Körper oder Körperstrukturen verglichen werden. Zum Beispiel kann in der vorliegenden Ausführungsform der erfasste Terahertz-Puls 32 Informationen über die Haut oder die Oberfläche oder das Gewebe der Hand 2 beinhalten und von der Recheneinheit 3 mit Informationen über bekanntes Gewebe, welche in der Datenbank 5 gespeichert sind, verglichen werden. Über eine Dateneingabevorrichtung 7, in der vorliegenden Ausführungsform ein Computertomograph 7, können Informationen über den Körper 2, insbesondere die Hand 2, erfasst werden, an die Recheneinheit 3 übertragen werden und in der Datenbank 5 als Referenzdaten zum Vergleich mit den erfassten Informationen gespeichert werden. Anstelle des in Figur 1 dargestellten Verfahrens der Ermittlung der Informationen über das Körperteil mittels Reflexion kann auch eine Untersuchung durch Transmission mittels der Strahlungsquelle 4 und des Terahertz-Sensors 1 oder durch Emission mittels des Terahertz-Sensors 1 erfolgen. Anstelle des gezeigten Computertomographen 7 kann auch eine andere Eingabevorrichtung, wie eine Tastatur, eine Röntgenvorrichtung wie ein C-Bogen, ein Ultraschall-Tomograph, ein Magnetresonanz-Tomograph, ein Positronen-Emissions-Tomograph oder ein SPECT-Tomograph verwendet werden um Informationen über ein Körperteil oder einen Körper 2 zu gewinnen und die Information in der Recheneinheit 3 auszuwerten oder in der Datenbank 5 als Referenzinformation zu speichern.

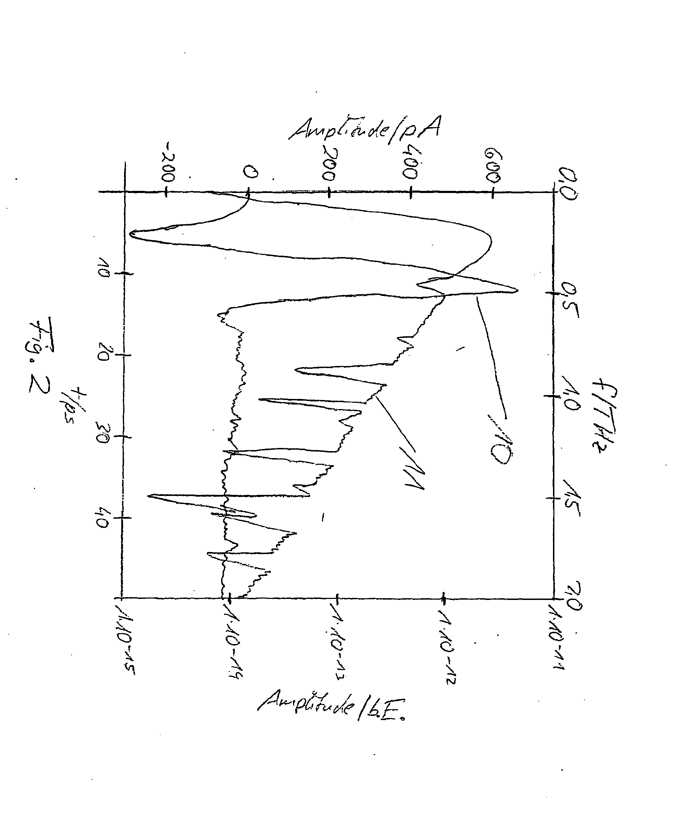

Figur 2 zeigt einen erfassten Terahertz-Puls im Zeitbereich 10 und im Frequenzbereich 11. Insbesondere die Frequenzbereichsdarstellung 11 des erfassten Terahertz-Signals kann von einer Recheneinheit mit Terahertz-Signalen bekannter Körperteile oder KörperStrukturen, wie Haut oder Gewebe, verglichen werden, um Rückschlüsse auf das untersuchte Körperteil oder die untersuchte Körperstruktur, wie das untersuchte Gewebe, zu ziehen. So kann z,B. die gesamte Frequenzbereichsdarstellung 11 mit gespeicherten Frequenzbereichsdarstellungen verglichen werden und durch das Auffinden des ähnlichsten Frequenzverlaufes kann auf die Art des untersuchten Gewebes geschlossen werden, wodurch z.B. gesundes von erkranktem Gewebe, wie Hautkrebs, unterschieden werden kann. Auch können charakteristische Frequenzen, wie die im Frequenzverlauf 11 zu erkennenden Frequenzen maximaler Absorption bzw. minimaler Reflektion mit in der Datenbank gespeicherten Frequenzen verschiedener Körperteile oder Körperstrukturen oder Gewebearten verglichen werden. Aus der größten Ähnlichkeit der Frequenzen maximaler Absorption bzw. minimaler Reflektion kann z.B. auf Eigenschaften, wie die Art oder die Zusammensetzung oder den Zustand der untersuchten Haut oder des untersuchten Gewebes geschlossen werden, wodurch insbesondere gesundes und krankes Gewebe unterschieden und erkannt werden kann.

## Patentansprüche

1. Verfahren zur Untersuchung eines Körpers (2), wobei die von dem Körper (2) ausgesandte und/oder reflektierte Strahlung im Teralzertz-Frequenzbereich, insbesondere zwischen 0,1 und 30 THz, mit einem Sensor (1) erfasst und von einer Recheneinheit (3) ausgewertet wird, um Informationen über den Körper (2) zu erhalten.

2. Verfahren zur Untersuchung eines Körpers (2) nach dem vorhergehenden Anspruch, wobei die mit dem Sensor (1) erfasste Strahlung so ausgewertet wird, dass Informationen über die Beschaffenheit, die Art, die Zusammensetzung, das Material, die Form, die Struktur, den Zustand, die Temperatur oder die Position der Oberfläche des Körpers (2) oder des Inneren des Körpers (2) erhalten werden.

3. Verfahren zur Untersuchung eines Körpers (2) nach einem der vorhergehenden Ansprüche, wobei basierend auf den Informationen über den Körper (2) oder die Körperstruktur, ein, insbesondere automatischer, Registrierungsvorgang des Körpers (2) oder der Körperstruktur durchgeführt wird.

4. Verfahren zur Untersuchung eines Körpers (2) nach einem der vorhergehenden Ansprüche, wobei die mittels der Terahertz-Strahlung ermittelten Informationen über den Körper (2) oder die Körperstruktur mittels eines Bildfusionsvorganges mit weiteren mittels der Terahertz-Strahlung oder eines weiteren bildgebenden Verfahrens gewonnenen oder bekannten Informationen über den Körper (2) oder die Körperstruktur kombiniert werden, um einen Gesamtdatensatz bestehend aus den mittels der Terahertz-Strahlung ermittelten Informationen über den Körper (2) oder die Körperstruktur und den weiteren mittels der Terahertz-Strahlung oder des weiteren bildgebenden Verfahrens gewonnenen oder bekannten Informationen über den Körper (2) oder die Körperstruktur zu erhalten.

5. Verfahren zur Untersuchung eines Körpers (2) nach dem vorhergehenden Anspruch, wobei als die weiteren bekannten Informationen über den Körper (2) oder die Körperstruktur ein zwei- und/oder dreidimensionaler Datensatz des Körpers (2) oder der Körperstruktur, insbesondere ein mittels eines Röntgenverfahrens, eines Magnetresonanz-Verfahrens, eines Computertomographie-Verfahrens, eines Ultraschall-Verfahrens, eines Positronen-Emissions-Tomographie-Verfahrens (PET-Verfahren) oder eines Single Photon Emission Computed Tomography Verfahrens (SPECT-Verfahren) ermittelter Datensatz, verwendet wird.

6. Verfahren zur Untersuchung eines Körpers (2) nach einem der vorhergehenden Ansprüche, wobei die Informationen über den Körper (2) oder die Körperstruktur mittels Terahertz-Strahlung durch Reflexion an dem Körper (2) oder der Körperstruktur oder durch Transmission durch den Körper (2) oder die Körperstruktur oder durch Emission des Körpers oder der Körperstruktur, insbesondere durch Kleidung hindurch, gewonnen werden,

7. Verfahren zur Untersuchung eines Körpers (2) nach einem der vorhergehenden Ansprüche, wobei basierend auf den Informationen über den Körper (2) oder die Körperstruktur, ein Navigationsvorgang, insbesondere die Navigation eines Instrumentes, durchgeführt wird.

8. Verfahren zur Untersuchung eines Körpers (2) nach einem der vorhergehenden Ansprüche, wobei die mit dem Sensor (1) erfasste Strahlung so ausgewertet wird, dass aus zeitlichen Informationen, insbesondere aus der Laufzeit der Strahlung, auf die Form, die Struktur, den Zustand oder die Position des Körpers (2) oder der Oberfläche des Körpers (2) oder der Körperstruktur geschlossen wird,

9. Verfahren zur Untersuchung eines Körpers (2) nach einem der vorhergehenden Ansprüche, wobei die mit dem Sensor (1) erfasste Strahlung so ausgewertet wird, dass spektrale Informationen über den Körper (2) oder die Körperstruktur im Terahertz-Bereich ermittelt werden, die ermittelten spektralen Informationen über den Körper (2) oder die Körperstruktur mit spektralen Informationen bekannter Körper (2) oder Körperstrukturen verglichen werden und aus dem durchgeführten Vergleich auf die Beschaffenheit, die Art, die Zusammensetzung, das Material, die Form, die Struktur, den Zustand, die Temperatur oder die Position der Oberfläche oder des Inneren des Körpers (2) oder der Körperstruktur geschlossen wird.

10. Verfahren zur Untersuchung eines Körpers (2) nach einem der vorhergehenden Ansprüche, wobei die mit dem Sensor (1) erfasste Strahlung so ausgewertet wird, dass charakteristischen Frequenzen, insbesondere Resonanzfrequenzen oder Absorptionsfrequenzen, des Körpers (2) oder der Körperstruktur ermittelt werden, die ermittelten charakteristischen Frequenzen mit charakteristischen Frequenzen, insbesondere mit Resonanzfrequenzen oder Absarptionsfrequerazen, bekannter Körper (2) oder Körperstrukturen verglichen werden und aus dem durchgeführten Vergleich auf die Beschaffenheit, die Art, die Zusammensetzung, das Material, die Form, die Struktur, den Zustand, die Temperatur oder die Position der Oberfläche oder des Inneren des Körpers (2) oder der Körperstruktur geschlossen wird.

11. Verfahren zur Untersuchung eines Körpers (2) nach einem der vorhergehenden Ansprüche, wobei als Körper (2) ein Kopf, ein Gesicht, ein Arm oder eine Hand untersucht wird.

12. Verfahren zur Untersuchung eines Körpers nach einem der vorhergehenden Ansprüche, wobei als die Körperstruktur Gewebe, Knochen bzw. Knochenstrukturen, Gefäße, Bänder, Sehnen, Zähne oder Haut eines Patienten untersucht werden.

13. Verfahren zur Untersuchung eines Körpers (2) nach einem der vorhergehenden Ansprüche, wobei der Körper (2) oder die Körperstruktur in einem Frequenzbereich zwischen 0,1 und 5 THz, insbesondere zwischen 0,1 und 0,6 THz oder zwischen 0,5 und 2 THz oder in einem Bereich um 1,6 THz oder in einem Bereich um 2,5 THz oder in einem Bereich um 3 THz untersucht wird.

14. Computerprogramm, welches, wenn es auf einem Computer läuft oder in einen Computer geladen ist, das Verfahren nach dem vorhergehenden Anspruch durchführt

15. Programmspeichermedium oder Computerprogrammprodukt mit dem Computerprogramm nach dem vorhergehenden Anspruch.

16. Vorrichtung zur Untersuchung eines Körpers (2) mit:
- einem Terahertz-Sensor (1), insbesondere einer Terahertz-Kamera, zum Erfassen von an einem Körper (2) oder einer Körperstruktur reflektierter oder durch einen Körper (2) oder eine Körperstruktur transmittierter oder von einem Körper (2) oder einer Körperstruktur emittierter Terahertz-Strahlung;
- einer Recheneinheit (3), wie einem Tracking-System, zum Auswerten der erfassten Terahertz-Strahlung, insbesondere zum Gewinnen von Informationen über den Körper (2) oder die Körperstruktur, wobei die Recheneinheit (3) mit dem Terahertz-Sensor (1) verbunden ist.

17. Vorrichtung zur Untersuchung eines Körpers (2) nach dem vorhergehenden Anspruch mit einer Terahertz-Strahlungsquelle (4), insbesondere Terahertz-Lampen, zum Aussenden von Terahertz-Strahlung auf den Körper (2) oder die Körperstruktur, wobei die Terahertz-Strahlungsquelle (4) mit dem Terahertz-Sensor (1) oder mit der Recheneinheit (3) verbunden sein kann, die ausgesandte Terahertz-Strahlung an dem Körper (2) oder der Körperstruktur reflektiert wird oder durch den Körper (2) oder die Körperstruktur transmittiert wird und von dem Terahertz-Sensor (1) erfasst wird.

18. Vorrichtung zur Untersuchung eines Körpers (2) nach einem der zwei vorhergehenden Ansprüche mit einer Datenbank (5), in welcher charakteristische Informationen einer Mehrzahl von Körpern (2) oder Körperstrukturen gespeichert sind, wobei die Datenbank (5) mit der Recheneinheit (3) verbunden ist, die Recheneinheit (3) einen Vergleich der mittels der Terahertz-Strahlung gewonnenen Informationen über den Körper (2) oder die Körperstruktur mit den charakteristischen Informationen der Mehrzahl von Körpern (2) oder Körperstrukturen durchführt, und basierend auf dem durchgeführten Vergleich Rückschlüsse auf die Beschaffenheit, die Art, die Zusammensetzung, das Material, die Form, die Struktur, den Zustand, die Temperatur oder die Position der Oberfläche oder des Inneren des Körpers (2) oder die Körperstruktur gezogen werden.

19. Vorrichtung zur Untersuchung eines Körpers (2) nach einem der drei vorhergehenden Ansprüche mit:
- einer Datenausgabevorrichtung (6), insbesondere einem Bildschirm, zur Ausgabe der Informationen über den Körper (2) die Körperstruktur;
- einer Dateneingabevorrichtung (7), insbesondere einer Tastatur oder einem Scanner, insbesondere einer Röntgenvorrichtung, einem Computertomographen, einem Kernspin-Tomographen, einem Ultraschall-Tomographen, einem Positronen-Emissians-Tamographen, einem SPECT-Tomographen, zur Eingabe von Informationen zur Abspeicherung in der Datenbank (5) oder von Informationen zur Verarbeitung in der Recheneinheit (3), insbesondere von charakteristischen Informationen über den Körper (2) oder die Körperstruktur.

20. Verfahren zur Untersuchung eines Körpers (2) nach einem der vier vorhergehenden Ansprüche, wobei die Terahertz-Strahlungsquelle (4) und/oder der Terahertz-Sensor (1) einen Terahertz-Oszillator, insbesondere einen Titan:Saphir-Laser, umfassen.
